# EUROPÄISCHE PATENTSCHRIFT

(11) **EP 0 857 036 B1**
(45) Veröffentlichungstag und Bekanntmachung des Hinweises auf die Patenterteilung: **15.09.2004**
(21) Anmeldenummer: 97930450.8
(22) Anmeldetag: 30.06.1997
(51) Int. Cl.: A61B 5/103, A61B 5/026

(54) **VORRICHTUNG ZUR VENÖSEN KOMPRESSIONSPLETHYSMOGRAPHIE**
DEVICE FOR CARRYING OUT A VENOUS PLETHYSMOGRAPHY USING COMPRESSION
DISPOSITIF POUR REALISER UNE PLETHYSMOGRAPHIE VEINEUSE A COMPRESSION

(30) Priorität: 09.08.1996 DE 19632263
(43) Veröffentlichungstag der Anmeldung: 12.08.1998
(73) Patentinhaber: TQ-Systems GmbH, 82229 Seefeld (DE)
(72) Erfinder: OSER, Daniel, D-82237 Woerthsee (DE); CHRIST, Frank, D-82166 Gräfelfing (DE)
(86) Internationale Anmeldenummer: PCT/EP1997/003409
(87) Internationale Veröffentlichungsnummer: WO 1998/006329

(56) Entgegenhaltungen:
- DE-C- 707 915
- US-A- 3 847 142
- US-A- 4 144 878
- US-A- 4 452 252

## Beschreibung

Die vorliegende Erfindung betrifft eine Vorrichtung zur venösen Kompressionsplethysmographie.

Die venöse Kompressionsplethysmographie ist ein bereits seit längerem bekanntes Verfahren zur Bestimmung mikrovaskulärer Parameter von Extremitäten, wie der venösen Kapazität, der venösen Elastizität, der venösen Abflußgeschwindigkeit, des arteriellen Blutflußes und der kapillären Filtrationsrate. Allgemein erlaubt die venöse Kompressionsplethysmographie qualitative und quantitative Aussagen über Zustand und Funktion der Mikrogefäße in einer Extremität eines Patienten.

Die venöse Kompressionsplethysmographie kann auf sehr unterschiedliche Weisen realisiert werden, zum Beispiel als Wasser-, Luft-, Impedanz-, Kapazitäts-, Induktions- oder Dehnungsmeßstreifenplethysmographie. Diese Verfahren verwenden unterschiedliche physikalische Phänomene, um den Zustand der Blutgefäße in einem Körperteil zu erfassen. Vorliegende Erfindung bezieht sich auf die Dehnungsmeßstreifenplethysmographie.

Bei der Dehnungsmeßstreifen- oder Strain-Gauge-Plethysmographie wird ein dehnbarer Meßstreifen um das zu untersuchende Körperteil, zum Beispiel einen Arm oder ein Bein gelegt, Herznäher wird in diesem Körperteil dann mit einer autblasbaren Manschette der venöse Rückfluß des Blutes behindert. Die Blutstauung führt zu einer Veränderung des Umfangs des betroffenen Körperteils, was wiederum zu einer Dehnung des Meßstreifens führt. Aus der Dehnung des Meßstreifens, die von dem auf die Manschette ausgeübten Druck abhängt, kann auf Eigenschaften oder Veränderungen der Blutgefäße geschlossen werden. Diese Auswertung der Dehnung in Abhängigkeit von der hervorgerufenen Stauung beruht auf bekannten Verfahren.

Aus der US 3 847 142 ist eine Vorrichtung zur Bestimmung des Blutflusses durch einen menschlichen Unterarm bekannt, die nach dem Prinzip der Dehnmeßstreifenplethysmographie funktioniert. Die Vorrichtung weist dabei eine Handgelenkmanschette auf, die zum vollständigen Unterbinden des Blutflusses zur Hand dient. Sie weist außerdem eine Oberarmmanschette auf, die sehr schnell bis zu einem Druck knapp unterhalb des diastolischen Blutdrucks aufgeblasen werden kann, um den venösen Ausfluss aus dem Arm zu unterbinden. Die Vorrichtung umfasst auch einen standardmäßigen Quecksilber-in-Gummi-Dehnmetistreifen, der um den größten Teil des Unterarms angeordnet wird, um Änderungen von dessen Umfang festzustellen. Der Dehnmeßstreifen bildet dabei eine Wheatstonebrückenschaltung, deren Ausgangsspannung sich linear mit der Länge des Dehnmebstreifens ändert. Die jeweiligen Änderungen der Dehnmeßstreifenlänge, und damit auch des Armumfangs, können dann von einer Logikschaltung in prozentuale Änderungen des Unterarmvolumens über die Zeit umgewandelt werden und in numerische Daten, die die Blutflussrate durch den Unterarm anzeigen. Um stets genaue Messwerte zu liefern, muss eine solche Vorrichtung vor der Benutzung kalibriert werden. Dies geschieht im hier beschriebenen Fall mittels einer Kalibrierschraube, die am Halte- bzw. Kalibrierbereich des Dehmeßstreifens angeordnet ist.

Typischerweise wird heute bei der Dehnungsmeßstreifenplethysmographie ein Meßstreifen verwendet, der aus einem mit Quecksilber gefüllten dehnbaren Silikonschlauch besteht. Bei einer Ausdehnung des Körperteils um den der Meßstreifen gelegt ist dehnt sich der Silikonschlauch aus und verformt die darin befindliche Quecksilbersäule. Dadurch ändert sich der elektrische Widerstand der Quecksilbersäule. Diese Widerstandsänderung wird gemessen und von ihr wird dann auf die Dehnung des Silikonschlauchs und damit des Körperteils zurückgeschlossen. Dieser Rückschluß setzt notwendigerweise die Kenntnis des Zusammenhangs zwischen der Widerstandsänderung und der Meßstreifendehnung voraus. Vorrichtungen zur Strain-Gauge-Plethysmographie müssen deshalb vor ihrem Einsatz kalibriert werden. Diese Kalibrierung muß praktisch vor jeder einzelnen Untersuchung eines Körperteils vorgenommen werden, da das Verhältnis von Widerstand der Quecksilbersäule zu Dehnung des Meßstreifens von einer Vielzahl von Parametern, wie der Umgebungstemperatur und der Körpertemperatur des Patienten, der Vorspannung des Silikonschlauchs und dem Umfang des untersuchten Körperteils abhängt.

Die Kalibrierung erfolgt dadurch, daß der in der Untersuchungsposition befestigte Meßstreifen definiert gedehnt wird und die dabei auftretende Widerstandsänderung gemessen wird. Typischerweise werden dabei mehrere aufeinanderfolgende definierte Dehnungen des Meßstreifens ausgeführt und die zugehörigen Widerstandsänderungen gemessen. Daraus wird dann unter Annahme eines linearen oder sonstigen Zusammenhangs der Meßgrößen ein Umrechnungsfaktor bestimmt. Zur Kalibrierung ist an dem Meßstreifen eine Kalibriervorrichtung angebracht, die in herkömmlichen Apparaten in der Regel aus einer Rändelschraube zur Dehnung des Meßstreifens besteht. Bei der Kalibrierung verstellt die Bedienperson, zum Beispiel ein Arzt oder eine Krankenschwester, dann die Länge des Meßstreifens immer wieder manuell.

Bei dieser manuellen Änderung der Dehnung übt die Bedienperson immer wieder Störkräfte auf das meist sehr leichte Meßsystem aus, so daß man für eine exakte Messung immer wieder die Relaxation des Systems abwarten muß. Die Relaxationszeiten des Systems können dabei sehr lang und sehr unterschiedlich sein. Eine zusätzliche Dehnung des Silikonschlauches zum Beispiel kann relativ schnell relaxieren, während eine Eindrückung im Gewebe des zu untersuchenden Körperteiles sehr viel mehr Zeit bis zum Wiedererreichen des Ausgangszustandes benötigt. Das führt zu erheblichen Zeitverlusten bei der Untersuchung. Außerdem besteht bei der manuellen Berührung der an dem Körperteil anlegenden Meßvorrichtung die Gefahr, daß der Meßstreifen auf der Haut des Patienten verschoben wird. Das kann zu Verfälschungen der Kalibrierung und damit zu Ungenauigkeiten bei der Auswertung der Meßwerte führen. Nachteilig ist insbesondere, daß es sehr vom Geschick der jeweiligen Bedienperson abhängt, ob die Messung schnell und zuverlässig durchgeführt werden kann. Dadurch verliert die Messung sowohl an Verläßlichkeit, wie auch an Reproduzierbarkeit. Um den durch die Bedienperson verursachten Meßfehler während einer Meßreihe unverändert zu halten, müßte nämlich die gleiche Bedienperson alle Messungen ausführen. Das ist gerade bei längeren Versuchsreihen und Forschungsprojekten nicht ohne weiteres möglich.

Aufgabe der Erfindung ist es deshalb, eine Vorrichtung zur Dehnungsmeßstreifenplethysmographie bereitzustellen, die schnell und einfach handhabbar ist und dabei aber zuverlässig genaue Meßwerte liefert. Diese Aufgabe wird durch die in den Vorrichtungsansprüchen beschriebene Apparatur gelöst.

Im Rahmen der vorliegenden Erfindung wird eine Vorrichtung zur venösen Kompressionsplethysmographie unter Verwendung eines Dehnungsmeßstreifens bereitgestellt, wobei der Dehnungsmeßstreifen ohne Berührung durch eine Bedienperson kalibriert wird. Vorzugsweise wird die Kalibrierung automotiv unter Verwendung eines Elektromotors, einer Pneumatik oder einer Federmechanik ausgeführt, der bzw. die für die Verstellung einer Rändelschraube oder sonstigen Vorschubeinrichtung sorgt, so daß die gewünschte definierte Dehnung eingestellt werden kann. Besonders bevorzugt ist die Verwendung eines Mikroprozessors zur Steuerung der Kalibriereinrichtung. Dadurch kann ohne direkten Kontakt der Bedienperson mit dem Meßstreifen oder der Kalibriereinrichtung die Kalibrierung der Dehnungsmessung vorgenommen werden.

Die erfindungsgemäße Vorrichtung wird im folgenden anhand der Figuren beschrieben. Dabei zeigt:
- Figur 1:: Prinzip der Dehnungsmeßstreifenplethysmographie.
- Figur 2:: Erfindungsgemäße Vorrichtung zur Dehnungsmeßstreifenplethysmographie.

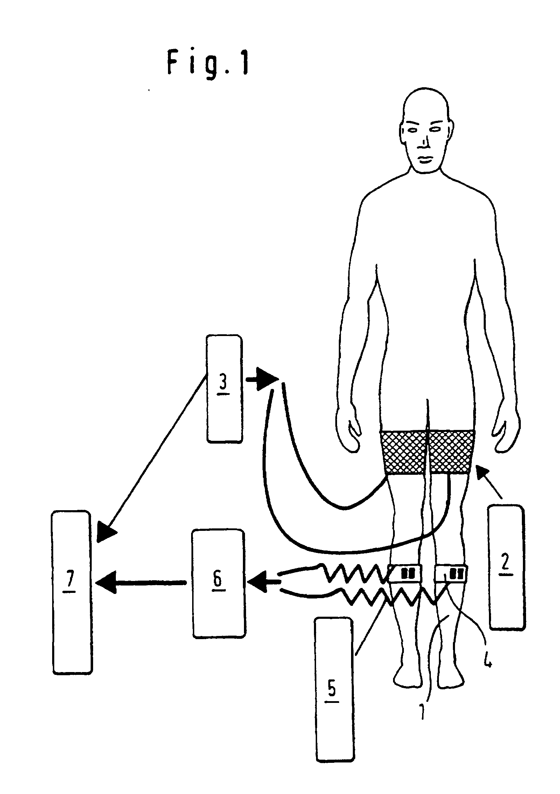

Figur 1 zeigt eine Vorrichtung zur venösen Kompressionsplethysmographie mit einer Manschette 2, die ein Körperteil 1, zum Beispiel ein Bein, umfaßt und deren Innendurchmesser I mit einer Pumpe 3 verändert werden kann. Weiterhin umfaßt die Vorrichtung einen Dehnungsmeßstreifen 4, der ebenfalls das Körperteil 1 umfaßt. Der Dehnungsmeßstreifen 4 besteht aus einem röhrenförmigen flexiblen Aufnahmematerial, vorzugsweise einem Silikonschlauch, und einem darin befindlichen dehnbaren und elektrisch leitenden Material, vorzugsweise Quecksilber oder einer Quecksilber enthaltenden Mischung. An beiden Enden des Meßstreifens 4 ist jeweils ein elektrischer Kontakt angebracht. Diese Kontakte sind über Leitungen 5 mit Aufzeichnungseinrichtung 6 verbunden, die zur Messung des elektrischen Widerstands in der Substanz oder einer dazu komplementären Größe wie dem Spannungsabfall oder dem Stromfluß geeignet ist. Die Signale der Aufzeichnungseinrichtung 6 und der Pumpe 3 werden dann zu einer Auswerteeinheit 7 geleitet, wo die Meßergebnisse in Abhängigkeit von einem Maß für die Änderung des Innendurchmessers der Manschette 2, im Regelfall dem von der Pumpe 3 auf die Manschette 2 ausgeübten Druck, ausgewertet werden.

Statt des elektrischen Widerstands kann auch eine beliebige andere Meßgröße M. zum Beispiel die Zahl von Interferenzlinien von sich überlagernden Lichtstrahlen, verwendet werden, deren Veränderung ΔM ein Maß für die Dehnung des Meßstreifens 4 ist. Der Dehnungsmeßstreifen kann dann entsprechend anders gestaltet sein, braucht also nicht aus einem Aufnahmematerial und einer darin angeordneten elektrisch leitenden Substanz zu bestehen.

Der Dehnungsmeßstreifen 4 muß auf dem Körperteil 1 in Umfangsrichtung beweglich sein, um sich bei einer Umfangsänderung des Körperteils 1 entlang seiner gesamten Länge dehnen zu können. Andernfalls kann der Dehnungsmeßstreifen 4 nämlich an einer bestimmten Stelle des Körperteils 1 haften bleiben. so daß er dort lokal überdehnt wird, während er an anderen Stellen überhaupt nicht gedehnt wird. Die Folge davon sind unzuverlässige Messungen. Im Regelfall wird dem Dehnungsmeßstreifen 4 deshalb ein Gleitband unterlegt, das eine Bewegung des Meßstreifens 4 entlang dem Umfang des Körperteils 1 erlaubt. Das kann dadurch erreicht werden, daß der Meßstreifen 4 auf dem Gleitband oder das Gleitband auf der Haut gleitet oder beide Effekte auftreten. Es ist aber auch denkbar, daß der Meßstreifen 4 selbst derart ausgebildet, zum Beispiel beschichtet ist, daß er auf der Haut gleitet.

An dem Dehnungsmeßstreifen 4 ist eine Kalibriervorrichtung angebracht. Im Rahmen der vorliegenden Erfindung erfolgt die Bedienung der Kalibriervorrichtung berührungsfrei, also ohne daß eine Bedienperson Hand anlegt. Das kann zum Beispiel mit Hilfe eines Elektromotors oder einer Pneumatik oder auch einer Federmechanik in der Kalibriervorrichtung geschehen. Die Kalibrierung wird vorzugsweise von einem Mikroprozessor gesteuert.

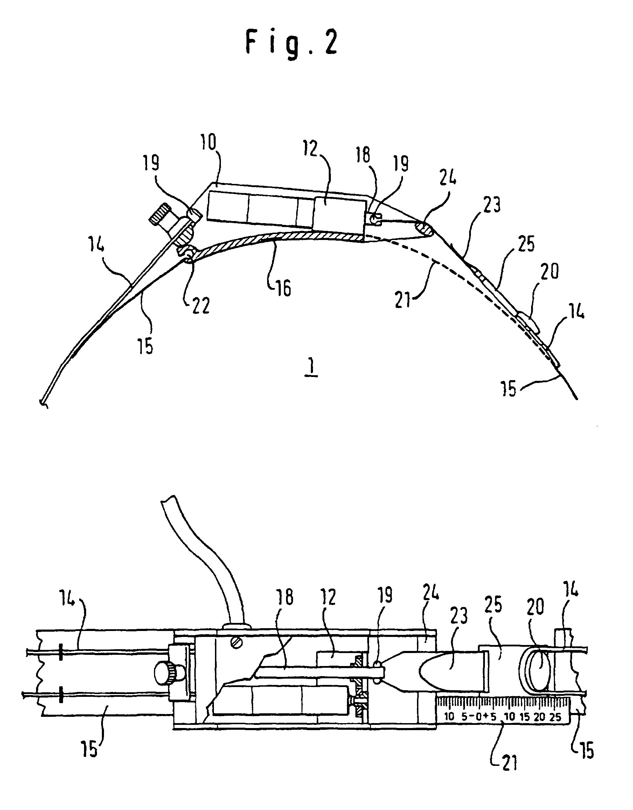

Figur 2 zeigt eine besonders bevorzugte Ausführungsform der erfindungsgemäßen Vorrichtung. Diese besitzt eine Kalibriervorrichtung 10 mit mindestens folgenden Elementen: Einem berührungsfrei zum Beispiel elektrisch oder pneumatisch gesteuerten Linearantrieb 12 mit einer Schubstange 18, die einen Befestigungspunkt 19 aufweist, und einem Spannband 23, das aus einem ersten Teil, der an Befestigungspunkt 19 mit der Schubstange 18 des Linearantriebs 12 verbunden ist, und einem zweiten Teil, der an einem Befestigungspunkt 20 mit dem Dehnungsmeßstreifen 14 verbunden ist, besteht, wobei der erste Teil als Schlaufe durch eine Lasche im zweiten Teil derart geführt ist, daß die Länge des Spannbands 23 zwischen den Befestigungspunkten 19 und 20 verändert werden kann. Dadurch kann die Meßvorrichtung auf Körperteilen unterschiedlichen Umfangs verwendet werden. Das andere Ende des Meßstreifens 14 ist an Klemmvorrichtung 13 mit der Kalibriervorrichtung 10 verbunden. Das Spannband 23 kann über eine Umlenkeinrichtung 24 geführt werden. um einen korrekten Verlauf sicherzustellen. Das Ende 25 des Spannbands 23 kann zum Beispiel mit einem Klettverschluß auf seinem gegenläufigen Teil fixiert sein. Weiter enthält die Kalibriervorrichtung 10 eine Längenmeßeinrichtung 21, die geeignet ist, das Körperteil 1 zu umfassen, aber auch nur aus einem relativ kleinen Abschnitt bestehen kann. Die Längenmeßeinrichtung ist derart angebracht, daß die Länge des Meßstreifens 14 bestimmt werden kann. Vorzugsweise weist die Kalibriervorrichtung 10 einen Befestigungspunkt 22 zur Befestigung eines das Körperteil 1 umfassenden Gleitbandes 15 auf, und, ebenfalls vorzugsweise, enthält sie einen Temperaturfühler 16 für die Messung der Oberflächentemperatur des Körperteils 1. Besonders bevorzugt ist die Anbringung eines weiteren, hier nicht gezeigten, Temperaturfühlers für die Umgebungstemperatur. Soweit technisch möglich kann natürlich auch ein einziger Temperaturfühler für die Bestimmung der Haut- und der Umgebungstemperatur verwendet werden. Es ist auch möglich, daß ein einziger Temperaturfühler eine Mischtemperatur aus Haut- und Umgebungstemperatur mißt, wenn diese Temperatur zur Korrektur der Meßwerte geeignet ist.

Die in Figur 1 gezeigte Vorrichtung wird folgendermaßen betrieben. Eine Manschette 2 wird um ein zu untersuchendes Körperteil 1 gelegt. Dabei kann es sich um eine größere Extremität wie ein Bein oder einen Arm handeln, es können aber auch kleinere Gliedmaßen wie Finger oder Zehen untersucht werden. Diese Manschette 2 erzeugt, wenn sie über Pumpe 3 entsprechend mit Druck versorgt wird, eine Stauung des venösen Blutrückstroms in dem dystal gelegenen Körperteil 1. Weiter vom Herzen entfernt wird auf demselben Körperteil dann ein Dehnungsmeßstreifen 4 angebracht. Wenn das Körperteil 1 aufgrund der erzeugten Blutabflußhemmung seinen Umfang verändert. dehnt sich auch der Meßstreifen 4 entsprechend. Dadurch ändert zum Beispiel die Substanz in dem Meßstreifen 4 ihren elektrischen Widerstand. Diese Widerstandsänderung wird mit einem Meßgerät 6, das über Leitung 5 an die beiden Enden des Meßstreifens 4 angeschlossen ist, gemessen. Um bei einem bestimmten Druck auf die Manschette 2, der ein Maß für deren Innendurchmesser I bzw. dessen Änderung ΔI ist, aus der Änderung ΔR des Widerstands R der Substanz auf eine Dehnung ΔD des Meßstreifens 4 zu schließen, muß die Apparatur zuerst kalibriert werden. Die Kalibrierung geschieht dadurch, daß mit Hilfe der zum Beispiel von einem Elektromotor angetriebenen Kalibriervorrichtung eine definierte Dehnung ΔD₁₂ eingestellt wird und der dazugehörige Wert der Widerstandsänderung ΔR₁₂ gemessen wird. Dieser Schritt kann mehrfach wiederholt werden, je nach den Anforderungen an die Meßgenauigkeit. Vorzugsweise wird die Kalibrierung durch Auswertung von zwei Widerstandsänderungen ΔR₁₂ und ΔR₂₃ für zwei aufeinanderfolgende definierte Dehnungen ΔD₁₂ und ΔD₂₃ vorgenommen. Bevorzugt ist aber auch eine Kalibrierung durch mindestens zweimalige Bestimmung der Widerstandsanderung ΔR₁₂ für die gleiche definierte Dehnung ΔD₁₂, wobei die Dehnung zwischen zwei Bestimmungen der Widerstandsänderung auf das Ausgangsmaß zurückgeführt wird.

Bei Verwendung der Vorrichtung nach Figur 2 wird zu Beginn der Messung der Meßstreifen 14 auf Gleitband 15 um das Körperteil 1 gelegt. Dazu wird der erste Teil des Spannbands 23 durch die Lasche des zweiten Teils des Spannbands 23 geführt und dann zum Beispiel mit einem Klettverschluß an dem gegenläufigen Teil des Spannbands 23 befestigt. Dann wird das Spannband 23 so eingestellt, daß der das Körperteil 1 umgebende dehnbare Meßstreifen 14 eine bestimmte vorgegebene Länge L₀ hat. Das ist möglich, weil der Meßstreifen 14 nicht das gesamte Körperteil 1 umfaßt. sondern durch die Kalibriervorrichtung 10 unterbrochen ist. Die Einstellung einer definierten effektiven Ausgangslänge L₀ des Meßstreifens 14 für die Messung ist nötig, weil die für eine relative Dehnung ΔD benötigte Kraft von der absoluten Länge bzw. der Vordehnung des Meßstreifens 14 abhängt. Unter effektiver Länge wird hier die an dem Körperteil 1 anliegende Länge des Dehnungsmeßstreifens 14 verstanden. Das an der Kalibriervorrichtung 10 angebrachte Längenmeßband 21. das das Körperteil 1 mindestens teilweise umgibt, ermöglicht die Bestimmung der effektiven Länge des Dehnungsmeßstreifens 14. Durch eine Verstellung der Länge des Spannbandes 23 kann die gewünschte Länge L₀ des Meßstreifens 14 eingestellt werden. Mit Hilfe des Längenmeßstreifens 21 kann auch der ungedehnte Umfang des Körperteils 1 in der Meßebene bestimmt werden. Wenn der Längenmeßstreifen 21 das Körperteil 1 vollständig umgibt, wird der Umfang des Körperteils direkt gemessen, wenn der Meßstreifen 21 das Körperteil 1 nur teilweise ergibt, muß die gewünschte Größe unter Zuhilfenahme der bekannten Länge zum Beispiel des Gleitbands 15 ermittelt werden.

Während der Messung sollte möglichst die Hauttemperatur des Körperteils I und die Umgebungstemperatur beobachtet werden. Eine Veränderung der Meßtemperatur um wenige Grad kann nämlich bereits zu einer Dehnung des Meßstreifens 14 führen, die zu einem signifikanten Meßfehler führt. Deshalb wird vorzugsweise ein Temperatursensor 16 für die Oberflächentemperatur des Körperteils 1 vorgesehen. Da die Meßtemperatur aber nicht unbedingt mit der Temperatur der Haut des Körperteils 1 übereinstimmen muß, kann auch ein weiterer Temperatursensor für die Umgebungstemperatur vorgesehen werden. Für beide Temperaturen oder für eine Mischtemperatur kann auch ein einziger Meßsensor vorgesehen werden. Entsprechend den Signalen der Temperatursensoren kann dann die Länge des Dehnungsmeßstreifens 14 nachgeregelt oder die Messung sonst korrigiert werden oder es kann ein Fehlersignal ausgegeben werden, das zum Abbruch der Messung führt.

Die erfindungsgemäße Vorrichtung bietet die Voraussetzung die Absolutwerte von mikrovaskulären Parametern und auch von deren periodischen Schwankungen einfach und zuverlässig zu bestimmen.

Im Hinblich auf weitere vorteilhafte Ausgestaltungen der Kalibriervorrichtung wird des weitere eine Vorrichtung zur venösen Kompressionsplethysmographie unter Verwendung einer direkten umfänglichen Längenänderung beschrieben wobei der induktive Wegmesser ohne Berührung durch eine Bedienperson kalibriert wird. Vorzugsweise wird die Kalibrierung automotiv unter Verwendung eines Elektromotors, einer Pneumatik oder einer Federmechanik ausgeführt, der bzw. die für die Verstellung einer Rändelschraube oder sonstigen Vorschubeinrichtung sorgt, so daß die gewünschte definierte Dehnung eingestellt werden kann. Besonders bevorzugt ist die Verwendung eines Mikroprozessors zur Steuerung der Kalibriereinrichtung. Dadurch kann ohne direkten Kontakt der Bedienperson mit dem Wegmesser oder der Kalibriereinrichtung die Kalibrierung der umfänglichen Längenänderung vorgenommen werden.

Die Vorrichtung wird im folgenden anhand der Figuren beschrieben. Dabei zeigt:

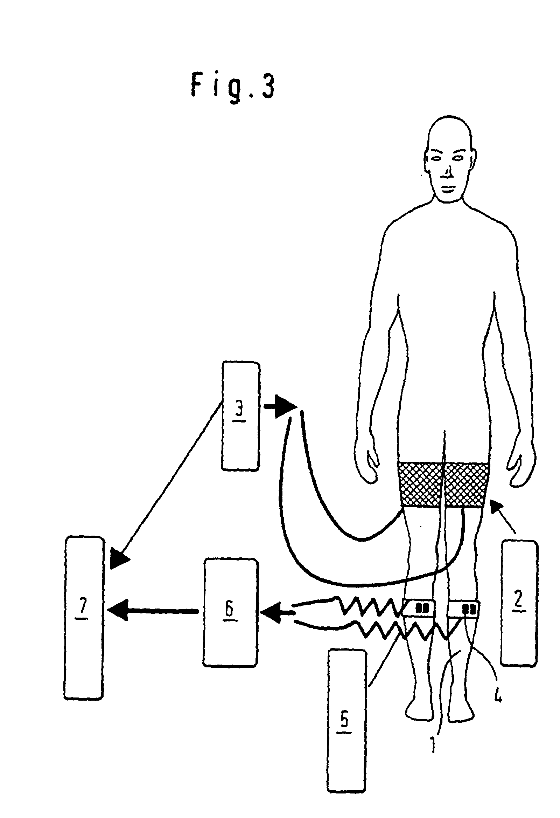

- Figur 3:: Prinzip der Plethysmographie.

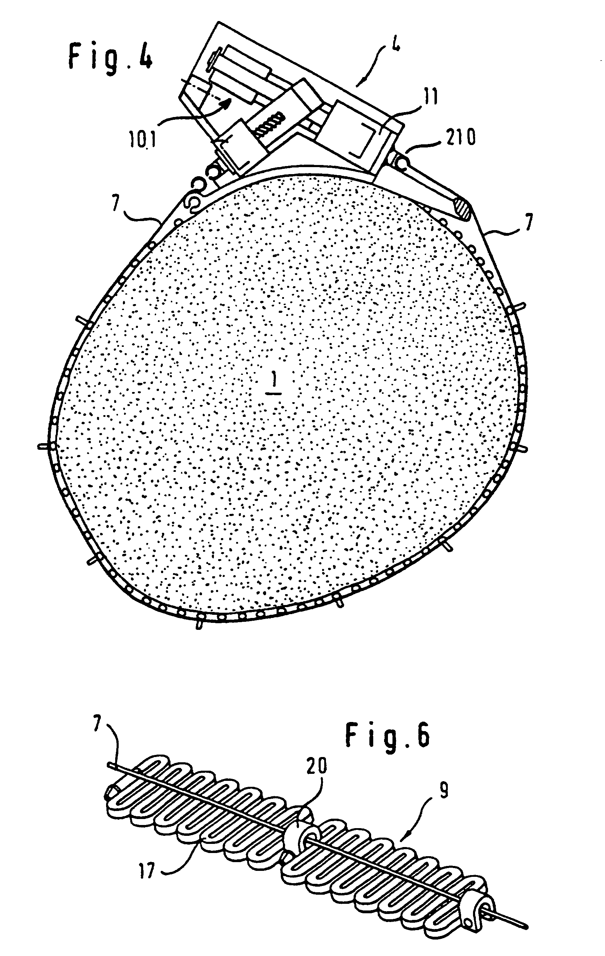

- Figur 4:: Vorrichtung zur Kompressionsplethysmographie.

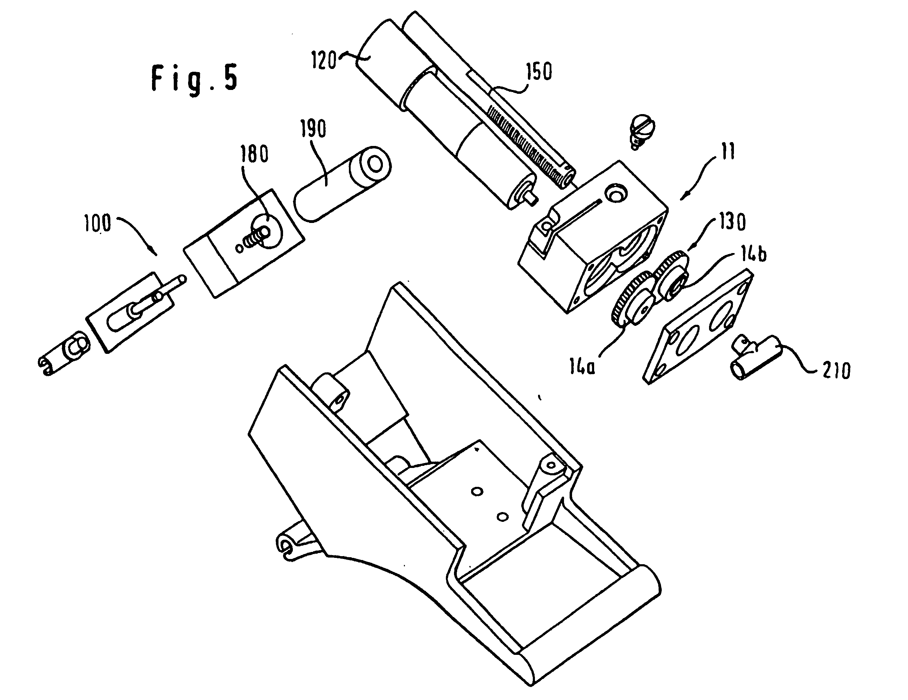

- Figur 5:: Explosionsdarstellung der Vorrichtung
- Figur 6:: Perspektivische Darstellung des Trägergurtes

Figur 3 zeigt eine Vorrichtung zur venösen Kompressionsplethysmographie mit einer Manschette 2, die ein Körperteil 1, zum Beispiel ein Bein, umfaßt und deren Innendurchmesser I mit einer Pumpe 3 verändert werden kann. Weiterhin umfaßt die Vorrichtung eine Meßanordnung 4, die ebenfalls das Körperteil 1 umfaßt. Die Meßanordnung 4 besteht aus einem bandförmigen Trägergurt, einer einstellbaren Lagereinrichtung 11, einem Wegmesser 100 sowie aus einem dehnungsarmen, forminstabilen Kraftübertragungselement 7.

Die über den Wegmesser gewonnenen Werte sind über Leitungen 5 mit Aufzeichnungseinrichtung 6 verbunden. Die Signale der Aufzeichnungseinrichtung 6 und der Pumpe 3 werden dann zu einer Auswerteeinheit 7 geleitet. wo die Meßergebnisse in Abhängigkeit von einem Maß für die Änderung des Innendurchmessers der Manschette 2, im Regelfall dem von der Pumpe 3 auf die Manschette 2 ausgeübten Druck, ausgewertet werden.

An der Meßanordnung 4 ist eine Kalibriervorrichtung angebracht. Die Bedienung der Kalibriervorrichtung erfolgt berührungsfrei, also ohne daß eine Bedienperson Hand anlegt. Das kann zum Beispiel mit Hilfe eines Elektromotors oder einer Pneumatik oder auch einer Federmechanik in der Kalibriervorrichtung geschehen. Die Kalibrierung wird vorzugsweise von einem Mikroprozessor gesteuert.

Figur 4 zeigt eine besonders bevorzugte Ausführungsform der Vorrichtung. Diese besitzt eine einstellbare Lagereinrichtung mit mindestens folgenden Elementen: Einem berührungsfrei zum Beispiel elektrisch oder pneumatisch gesteuerten Stellantrieb 101 mit einer Schubstange, einer Getriebestufe, die aus zwei Stirnrädern besteht und einem Kupplungsstück 210, an dem das dehnungsarme forminstabile Kraftübertragungselement angekuppelt werden kann.

Figur 5 zeigt eine Explosionsdarstellung der einzelnen Baugruppen der erfindungsgemäßen Meßanordnung 4. Der Wegmesser 100 der erfindungsgemäßen Vorrichtung besteht aus einem Zylinder 190 der in einer Bohrung 180 bewegt wird. Durch die Bewegung wird eine Induktionsspannung induziert, so daß der zurückgelegte Weg mit der induzierten Spannung in Relation steht.

Ebenso ist eine andere Ausgestaltung des Wegmessers möglich, so z.B. piezoelektronische Sensoren, die ein entsprechendes Signal liefern können, oder optische Wegmesser, die z.B. mittels Inkrementalgeber Wegstrecken messen können.

Eine andere Ausführungsform integriert die Lagereinrichtung und den Wegmesser derart, daß zum Kalibrieren nur noch ein Signal abgefragt werden muß, nämlich die Induktionsspannung im Verhältnis zur Spindelhöhe. Dies kann z.B. in einem piezoelektronischen Stellantrieb realisiert werden.

Die Lagereinrichtung 11 weist einen elektrischen Stellantrieb 120 auf, dem eine Getriebestufe 130 nachgeschaltet ist. Die Getriebestufe 130 weist ein Stirnrad 14a an der Frontseite des elektrischen Stellantriebs 120 und ein Stirnrad 14b an der Frontseite einer Spindelstange 150 auf, wobei die Nabe des Stirnrades 14b offen gestaltet ist, und das dehnungsarme forminstabilen Kraftübertragungselement 7 mit der Spindelstange lösbar verbunden ist. Der Stellmotor 120 treibt die Getriebestufe 130 an, wobei die Spindelstange 15 durch ein Innengewinde in Stirnrad 14b eine Linearbewegung ausführt. Diese Linearbewegung vermag das dehnungsarme forminstabile Kraftübertragungselement mittels Kupplungsstück 210 zu spannen oder zu lockern, so daß ein kontrolliertes Nachführen des Kraftübertragungselement erfolgen kann. Die in Figur 3 gezeigte Vorrichtung wird folgendermaßen betrieben. Eine Manschette 2 wird um ein zu untersuchendes Körperteil 1 gelegt. Dabei kann es sich um eine größere Extremität wie ein Bein oder einen Arm handeln, es können aber auch kleinere Gliedmaßen wie Finger oder Zehen untersucht werden. Diese Manschette 2 erzeugt, wenn sie über Pumpe 3 entsprechend mit Druck versorgt wird, eine Stauung des venösen Blutrückstroms in dem distal gelegenen Körperteil 1. Weiter vom Herzen entfernt wird auf demselben Körperteil dann eine Meßanordnung 4 angebracht. Wenn das Körperteil 1 aufgrund der erzeugten Blutabflußhemmung seinen Umfang verändert, wird der Trägergurt gedehnt, was eine Auslenkung des Kraftübertragungselement im Wegmesser zur Folge, wodurch eine Spannung induziert wird. Diese Spannungsänderung wird mit einem Meßgerät 6. das über Leitung 5 an die beiden Enden des Meßstreifens 4 angeschlossen ist, gemessen. Um bei einem bestimmten Druck auf die Manschette 2. der ein Maß für deren Innendurchmesser I bzw. dessen Änderung ΔV ist, aus der Änderung ΔV des Wegmessers auf eine Längenänderung ΔL des Kraftübertragungselements zu schließen muß die Apparatur zuerst kalibriert werden. Die Kalibrierung geschieht dadurch, daß mit Hilfe der zum Beispiel von einem Elektromotor angetriebenen Kalibriervorrichtung eine definierte Dehnung ΔL₁₂ eingestellt wird und der dazugehörige Wert der Widerstandsänderung ΔV₁₂ gemessen wird. Dieser Schritt kann mehrfach wiederholt werden, je nach den Anforderungen an die Meßgenauigkeit. Vorzugsweise wird die Kalibrierung durch Auswertung von zwei Spannungsänderungen ΔV₁₂ und ΔV₂₃ für zwei aufeinanderfolgende definierte Dehnungen ΔL₁₂ und ΔL₂₃ vorgenommen. Bevorzugt ist aber auch eine Kalibrierung durch mindestens zweimalige Bestimmung der Spannungsänderung ΔV₁₂ für die gleiche definierte Dehnung ΔL₁₂, wobei die Dehnung zwischen zwei Bestimmungen der Spannungsänderung auf das Ausgangsmaß zurückgeführt wird.

Bei Verwendung der Vorrichtung nach Figur 4 wird zu Beginn der Messung der Trägergurt um das Körperteil 1 gelegt. Die Länge des Trägergurtes kann in Abhängigkeit des Umfanges der Extremität variiert werden indem einzelne Elemente modular miteinander verbunden werde. Vorzugsweise geschieht dies durch Schnappverbindungen, die sowohl einen sicheren Halt als auch eine zuverlässige Lösbarkeit bieten. Nachdem die Meßanordnung in gewünschter Weise auf der Extremität plaziert worden ist, wird die Kalibrierung durchgeführt.

Figur 6 zeigt den bandartigen Trägergurt, der als Auflage auf der Haut der Extremität 1 dient und so ausgebildet ist, daß er im wesentlichen durch Haftung an der Hautoberfläche einen zuverlässigen Halt bietet, so daß ein Verrutschen der Meßanordung vermieden werden kann. Der Trägergurt weist vorzugsweise einen mäanderförmigen Querschnitt auf, der sich in Längsrichtung erstreckt. Durch diese mäanderförmige Gestaltung kann der Trägergurt bei Zugbelastung in Längsrichtung gedehnt werden, und so die an der Oberseite befindlichen Führungsvorrichtungen mitführen. Die Führung der Kraftübertragungselemente ist somit jederzeit zuverlässig und im wesentlichen reibungsfrei gewährleistet. Der Trägergurt weist ferner modulare Einzetelemente auf, die durch eine lösbare Schnappverbindung derart miteinander verbindbar sind, daß die Länge des Trägergurtes nach Belieben einstellbar ist, um den Gegebenheiten unterschiedlicher Extremitäten gerecht zu werden.

Die modularen Einzelelemente weisen für die sichere und zuverlässige Führung des Kraftübertragungselemente Vorrichtungen auf, die vorzugsweise ringförmig ausgebildet sind.

Das dehnungsarme, forminstabile Kraftübertragungselement ist vorzugsweise eine Garn aus einem Polyesterwerkstoff, der sich ferner durch eine glatte, und somit reibungsarme Oberfläche auszeichnet. Ebenso sind andere Werkstoffe für die Ausbildung des Kraftübertragungselement möglich, so z.B. Polyamidgarne oder Kohlefasern.

Die Vorrichtung bietet die Voraussetzung die Absolutwerte von mikrovaskulären Parametern und auch von deren periodischen Schwankungen einfach und zuverlässig zu bestimmen.

## Patentansprüche

1. Vorrichtung zur venösen Kompressionsplethysmographie mit einer Manschette (2), deren Innendurchmesser I veränderbar ist und die geeignet ist, ein Körperteil (1) zu umfassen,
mit einem Dehnungsmeßstreifen (4), der geeignet ist das Körperteil (1) zu umfassen, und
einer Kalibriervorrichtung, die mit dem Dehnungsmeßstreifen (4) verbunden ist und eine definierte Dehnung ΔD₁₂ des Meßstreifens (4) erlaubt,
**dadurch gekennzeichnet, daß**
die Kalibriervorrichtung einen Verstellmechanismus aufweist, der die definierte Dehnung ΔD₁₂ ohne Berührung des Dehnungsmeßstreifens (4) oder der Kalibriervorrichtung durch eine Bedienperson erzeugen kann.

2. Vorrichtung nach Anspruch 1, **dadurch gekennzeichnet, daß** der Dehnungsmeßstreifen (4) aus einem Aufnahmematerial und einer darin befindlichen elektrisch leitenden Substanz S besteht und Kontakte zur Messung des elektrischen Widerstands R in der Substanz S aufweist.

3. Vorrichtung nach Anspruch 1 oder 2, **dadurch gekennzeichnet, daß** das Aufnahmematerial ein Silikonschlauch und/oder die Substanz S Quecksilber oder eine Quecksilber enthaltende Mischung ist.

4. Vorrichtung nach einem der Ansprüche 1 bis 3, **dadurch gekennzeichnet, daß** die Kalibriervorrichtung einen Elektromotor oder eine Pneumatik zur Erzeugung der definierten Dehnung ΔD₁₂ enthält.

5. Vorrichtung nach einem der Ansprüche 1 bis 4, **gekennzeichnet durch** einen Mikroprozessor zur Steuerung der Kalibrierung.

6. Vorrichtung nach einem der Ansprüche 1 bis 5, **gekennzeichnet durch** eine Kalibriervorrichtung (10) mit mindestens folgenden Elementen:
einem berührungsfrei gesteuerten Linearantrieb (12) mit einer Schubstange (18), die einen Befestigungspunkt (19) aufweist,
einem Spannband (23), das aus einem ersten Teil, der an Befestigungspunkt (19) mit der Schubstange (18) des Linearantriebs (12) verbunden ist und einem zweiten Teil, der an einem Befestigungspunkt (20) mit dem Dehnungsmeßstreifen (14) verbunden ist, besteht, wobei der erste Teil als Schlaufe **durch** eine Lasche im zweiten Teil derart geführt ist, daß die Länge des Spannbands (23) zwischen den Befestigungspunkten (19) und (20) verändert werden kann,
einer Längenmeßeinrichtung (21), die das Körperteil (1) umfassen kann und
derart angebracht ist, daß die effektive Länge des Dehnungsmeßstreifens (14) und der Umfang des Körperteils (1) in der Meßebene bestimmt werden kann.

7. Vorrichtung nach einem der Ansprüche 1 bis 6, **gekennzeichnet durch** einen Befestigungspunkt (22) zur Befestigung eines das Körperteil (1) umfassenden Gleitbandes (15).

8. Vorrichtung nach einem der Ansprüche 1 bis 7, **gekennzeichnet durch** einen Temperaturfühler (16) für die Messung der Oberflächentemperatur und/oder der Umgebungstemperatur des Körperteils (1).

## Claims

1. A device for use in venous compression plethysmography, comprising a cuff (2) the inside diameter *l* of which is variable and suitable to enclose an extremity or part (1);
a strain gauge foil (4) suitable to enclose extremity or part (1); and
a calibrator being in communication with strain gauge foil (4) and permitting a defined strain ΔD₁₂ of gauge (4),
**characterized in that**
the calibrator includes an adjusting mechanism capable to generate the defined strain ΔD₁₂ with no contact on the part of the operator with the strain gauge foil (4) or the calibrator.

2. A device according to claim 1, **characterized in that** the strain gauge foil (4) is composed of an accommodating material and an electrically conductive substance S contained therein, and of contacts for measuring the electric resistance R in substance S.

3. A device according to claims 1 or 2, **characterized in that** the accommodating material is a silicon tube and/or substance S is mercury or a mercury-containing mix.

4. A device according to any one of claims 1 through 3, **characterized in that** the calibrator contains an electromotor or a pneumatic system for generating the defined strain ΔD₁₂.

5. A device according to any one of claims 1 through 4, **characterized by** a micro processor for controlling the calibration process.

6. A device according to any one of claims 1 through 5, **characterized by** a calibrator (10) including at least the following elements:
a contact-free controlled linear drive (12) having a push bar (18) provided with a point of attachment (19);
a stress band (23) composed of a first portion connected at the point of attachment (19) to push bar (18) of the lines drive (12), and a second portion connected at a point of
attachment (20) to the strain gauge (14), with the first portion being guided as a loop through a flap within the second portion such that the length of the stress band (23) between the points of attachment (19) and (20) can be varied;
a length measuring means (21) capable to enclose the extremity or part (1) and being so mounted as to determine in the plane of measurement the effective length of the strain gauge (14) and the volume of part (1).

7. A device according to any one of claims 1 through 6, **characterized by** a point of attachment (22) for fixing a slide tape (15) enclosing the extremity or part (1).

8. A device according to any one of claims 1 through 7, **characterized by** a temperature sensor (16) for measuring the surface temperature and /or the ambient temperature of the extremity or part (1).

## Revendications

1. Dispositif pour la pléthysmographie de compression veineuse avec une manchette (2) dont le diamètre intérieur I peut être modifié et qui convient à entourer une partie du corps (1), avec une jauge de contrainte (4) convenant à entourer la partie du corps (1) et avec un dispositif de calibrage relié à la jauge de contrainte (4) et qui permet une extension définie ΔD₁₂ de la jauge de contrainte, **caractérisé en ce que** le dispositif de calibrage est pourvu d'un mécanisme de réglage qui peut générer l'extension définie ΔD₁₂ sans que l'opérateur touche la jauge de contrainte (4) ou le dispositif de calibrage.

2. Dispositif selon la revendication 1, **caractérisé en ce que** la jauge de contrainte (4) est constituée d'un matériau de réception et d'une substance S électro-conductrice qui se trouve dans son intérieur et qui est pourvue de contacts pour la mesure de la résistance électrique R dans la substance S.

3. Dispositif selon la revendication 1 ou 2, **caractérisé en ce que** le matériau de réception est un tuyau flexible en silicone et/ou que la substance S est du mercure ou un mélange contenant du mercure.

4. Dispositif selon l'une des revendications 1 à 3, **caractérisé en ce que** le dispositif de calibrage contient un moteur électrique ou une pneumatique pour la génération de l'extension ΔD₁₂ définie.

5. Dispositif selon l'une des revendications 1 à 4, **caractérisé par** un microprocesseur pour la commande du calibrage.

6. Dispositif selon l'une des revendications 1 à 5, **caractérisé par** un dispositif de calibrage (10) avec les éléments suivants au minimum :
entraînement linéaire (12) commandé sans contact avec une tige de poussée (18) qui dispose d'un point de fixation (19),
bande de tension (23) qui est constituée d'une première partie qui est reliée au point de fixation (19) par la tige de poussée (18) de l'entraînement linéaire (12) et
d'une deuxième partie qui est reliée au point de fixation (20) avec la jauge de contrainte (14), la première partie étant réalisée comme boucle pouvant être passée par une languette dans la deuxième partie de manière à ce que la longueur de la bande de tension (23) entre les points de fixation (19) et (20) puisse être modifiée,
dispositif de mesure de longueur (21) qui peut entourer la partie de corps (1) et
qui est disposé de manière à ce que la longueur effective de la jauge de contrainte (14) et le tour de la partie du corps (1) puissent être déterminés dans le niveau de mesure.

7. Dispositif selon l'une des revendications 1 à 6, **caractérisé par** un point de fixation (22) servant à la fixation d'une bande de glissement (15) entourant la partie du corps (1).

8. Dispositif selon l'une des revendications 1 à 7, **caractérisé par** une sonde de température (16) pour la mesure de la température de surface et/ou de la température ambiante de la partie de corps (1).
